# EUROPEAN PATENT APPLICATION

(11) **EP 4 714 347 A1**
(43) Date of publication of application: **25.03.2026**
(21) Application number: 25208854.7
(22) Date of filing: 11.03.2021
(51) Int. Cl.: A61B 5/145, A61B 5/00, G16H 15/00, G16H 50/30, A61B 5/1468, G01N 33/487

(54) **GRAPHICAL USER INTERFACES FOR ANALYTE MONITORING SYSTEMS**

(30) Priority: 11.03.2020 US 202062988326 P
(62) Divisional of application: 25188115.7
(71) Applicant: Abbott Diabetes Care Inc., Alameda, CA 94502 (US)
(72) Inventor: KUMAR, Panganamala Ashwin, California, 94618 (US); WOO, Jennifer, California, 94610 (US); ROSSI, Stephen A., California, 94517 (US); JANGAM, Sujit, California, 94403 (US); COVINGTON, Kendall, California, 94609 (US); LANG, Jordan Wing-Haye, California, 95133 (US)
(74) Representative: Mathys & Squire

(57) **Abstract**

Improved graphical user interfaces for analyte monitoring systems are provided herein. In particular, disclosed herein are various embodiments of Time-in-Ranges interfaces and Analyte Level/Trend Alert interfaces.

## Description

### FIELD

The subject matter described herein relates generally to graphical user interfaces for analyte monitoring systems, as well as methods and devices relating thereto.

### BACKGROUND

The detection and/or monitoring of analyte levels, such as glucose, ketones, lactate, oxygen, hemoglobin A1C, or the like, can be vitally important to the health of an individual having diabetes. Patients suffering from diabetes mellitus can experience complications including loss of consciousness, cardiovascular disease, retinopathy, neuropathy, and nephropathy. Diabetics are generally required to monitor their glucose levels to ensure that they are being maintained within a clinically safe range, and may also use this information to determine if and/or when insulin is needed to reduce glucose levels in their bodies, or when additional glucose is needed to raise the level of glucose in their bodies.

Growing clinical data demonstrates a strong correlation between the frequency of glucose monitoring and glycemic control. Despite such correlation, however, many individuals diagnosed with a diabetic condition do not monitor their glucose levels as frequently as they should due to a combination of factors including convenience, testing discretion, pain associated with glucose testing, and cost.

To increase patient adherence to a plan of frequent glucose monitoring, in vivo analyte monitoring systems can be utilized, in which a sensor control device may be worn on the body of an individual who requires analyte monitoring. To increase comfort and convenience for the individual, the sensor control device may have a small form-factor and can be applied by the individual with a sensor applicator. The application process includes inserting at least a portion of a sensor that senses a user's analyte level in a bodily fluid located in a layer of the human body, using an applicator or insertion mechanism, such that the sensor comes into contact with a bodily fluid. The sensor control device may also be configured to transmit analyte data to another device, from which the individual or her health care provider ("HCP") can review the data and make therapy decisions.

Despite their advantages, however, some people are reluctant to use analyte monitoring systems for various reasons, including the complexity and volume of data presented, a learning curve associated with the software and user interfaces for analyte monitoring systems, and an overall paucity of actionable information presented.

Thus, needs exist for graphical user interfaces for analyte monitoring systems, as well as methods and devices relating thereto, that are robust, user-friendly, and provides for timely and actionable responses.

### SUMMARY

Provided herein are example embodiments of graphical user interfaces ("GUIs") for in vivo analyte monitoring systems. According to some embodiments, a Time-in-Ranges ("TIR") GUI of an analyte monitoring system is provided, wherein the TIR GUI comprises a plurality of bars or bar portions, wherein each bar or bar portion indicates an amount of time that a user's analyte level is within a predefined analyte range correlating with the bar or bar portion. In some embodiments, for example, the amount of time can be expressed as a percentage of total time. According to another embodiment, an Analyte Level/Trend Alert GUI of an analyte monitoring system is provided, wherein the Analyte Level/Trend Alert GUI comprises a visual notification (e.g., alert, alarm, pop-up window, banner notification, etc.), wherein the visual notification includes an alarm condition, an analyte level measurement associated with the alarm condition, and a trend indicator associated with the alarm condition. In some embodiments, for example, the trend indicator comprises a directional trend arrow.

The embodiments provided herein are improved GUIs or GUI features for analyte monitoring systems that are highly intuitive, user-friendly, and provide for rapid access to physiological information of a user. More specifically, these embodiments allow a user to easily navigate through and between different user interfaces that can quickly indicate to the user various physiological conditions and/or actionable responses, without requiring the user (or an HCP) to go through the arduous task of examining large volumes of analyte data. Other improvements and advantages are provided as well. The various configurations of these devices are described in detail by way of the embodiments which are only examples.

Other systems, devices, methods, features and advantages of the subject matter described herein will be or will become apparent to one with skill in the art upon examination of the following figures and detailed description. It is intended that all such additional systems, devices, methods, features, and advantages be included within this description, be within the scope of the subject matter described herein, and be protected by the accompanying claims. In no way should the features of the example embodiments be construed as limiting the appended claims, absent express recitation of those features in the claims.

### BRIEF DESCRIPTION OF THE FIGURES

The details of the subject matter set forth herein, both as to its structure and operation, may be apparent by study of the accompanying figures, in which like reference numerals refer to like parts. The components in the figures are not necessarily to scale, emphasis instead being placed upon illustrating the principles of the subject matter. Moreover, all illustrations are intended to convey concepts, where relative sizes, shapes and other detailed attributes may be illustrated schematically rather than literally or precisely.
FIG. 1 is a system overview of a sensor applicator, reader device, monitoring system, network, and remote system.
FIG. 2A is a block diagram depicting an example embodiment of a reader device.
FIGS. 2B and 2C are block diagrams depicting example embodiments of sensor control devices.
FIGS. 3A to 3F are example embodiments of GUIs comprising time-in-ranges interfaces.
FIGS. 4A to 4O are example embodiments of GUIs comprising analyte level and trend alert interfaces.

### DETAILED DESCRIPTION

Before the present subject matter is described in detail, it is to be understood that this disclosure is not limited to the particular embodiments described, as such may, of course, vary. It is also to be understood that the terminology used herein is for the purpose of describing particular embodiments only, and is not intended to be limiting, since the scope of the present disclosure will be limited only by the appended claims.

As used herein and in the appended claims, the singular forms "a," "an," and "the" include plural referents unless the context clearly dictates otherwise.

The publications discussed herein are provided solely for their disclosure prior to the filing date of the present application. Nothing herein is to be construed as an admission that the present disclosure is not entitled to antedate such publication by virtue of prior disclosure. Further, the dates of publication provided may be different from the actual publication dates which may need to be independently confirmed.

Generally, embodiments of the present disclosure include GUIs for analyte monitoring systems, and methods and devices relating thereto. Accordingly, many embodiments include in vivo analyte sensors structurally configured so that at least a portion of the sensor is, or can be, positioned in the body of a user to obtain information about at least one analyte of the body. It should be noted, however, that the embodiments disclosed herein can be used with in vivo analyte monitoring systems that incorporate in vitro capability, as well as purely in vitro or ex vivo analyte monitoring systems, including systems that are entirely non-invasive.

Furthermore, for each and every embodiment of a method disclosed herein, systems and devices capable of performing each of those embodiments are covered within the scope of the present disclosure. For example, embodiments of sensor control devices, reader devices, local computer systems, and trusted computer systems are disclosed, and these devices and systems can have one or more sensors, analyte monitoring circuits (e.g., an analog circuit), memories (e.g., for storing instructions), power sources, communication circuits, transmitters, receivers, processors and/or controllers (e.g., for executing instructions) that can perform any and all method steps or facilitate the execution of any and all method steps.

As previously described, a number of embodiments described herein provide for improved GUIs for analyte monitoring systems, wherein the GUIs are highly intuitive, user-friendly, and provide for rapid access to physiological information of a user. According to some embodiments, a Time-in-Ranges GUI of an analyte monitoring system is provided, wherein the Time-in-Ranges GUI comprises a plurality of bars or bar portions, wherein each bar or bar portion indicates an amount of time that a user's analyte level is within a predefined analyte range correlating with the bar or bar portion. According to another embodiment, an Analyte Level/Trend Alert GUI of an analyte monitoring system is provided, wherein the Analyte Level/Trend Alert GUI comprises a visual notification (e.g., alert, alarm, pop-up window, banner notification, etc.), and wherein the visual notification includes an alarm condition, an analyte level measurement associated with the alarm condition, and a trend indicator associated with the alarm condition. In sum, these embodiments provide for a robust, user-friendly interfaces that can increase user engagement with the analyte monitoring system and provide for timely and actionable responses by the user, to name a few advantages.

Before describing these aspects of the embodiments in detail, however, it is first desirable to describe examples of devices that can be present within, for example, an in vivo analyte monitoring system, as well as examples of their operation, all of which can be used with the embodiments described herein.

There are various types of in vivo analyte monitoring systems. "Continuous Analyte Monitoring" systems (or "Continuous Glucose Monitoring" systems), for example, can transmit data from a sensor control device to a reader device continuously without prompting, e.g., automatically according to a schedule. "Flash Analyte Monitoring" systems (or "Flash Glucose Monitoring" systems or simply "Flash" systems), as another example, can transfer data from a sensor control device in response to a scan or request for data by a reader device, such as with a Near Field Communication (NFC) or Radio Frequency Identification (RFID) protocol. In vivo analyte monitoring systems can also operate without the need for finger stick calibration.

In vivo analyte monitoring systems can be differentiated from "in vitro" systems that contact a biological sample outside of the body (or "ex vivo") and that typically include a meter device that has a port for receiving an analyte test strip carrying bodily fluid of the user, which can be analyzed to determine the user's blood sugar level.

In vivo monitoring systems can include a sensor that, while positioned in vivo, makes contact with the bodily fluid of the user and senses the analyte levels contained therein. The sensor can be part of the sensor control device that resides on the body of the user and contains the electronics and power supply that enable and control the analyte sensing. The sensor control device, and variations thereof, can also be referred to as a "sensor control unit," an "on-body electronics" device or unit, an "on-body" device or unit, or a "sensor data communication" device or unit, to name a few.

In vivo monitoring systems can also include a device that receives sensed analyte data from the sensor control device and processes and/or displays that sensed analyte data, in any number of forms, to the user. This device, and variations thereof, can be referred to as a "handheld reader device," "reader device" (or simply a "reader"), "handheld electronics" (or simply a "handheld"), a "portable data processing" device or unit, a "data receiver," a "receiver" device or unit (or simply a "receiver"), or a "remote" device or unit, to name a few. Other devices such as personal computers have also been utilized with or incorporated into in vivo and in vitro monitoring systems.

### Example Embodiment of In Vivo Analyte Monitoring System

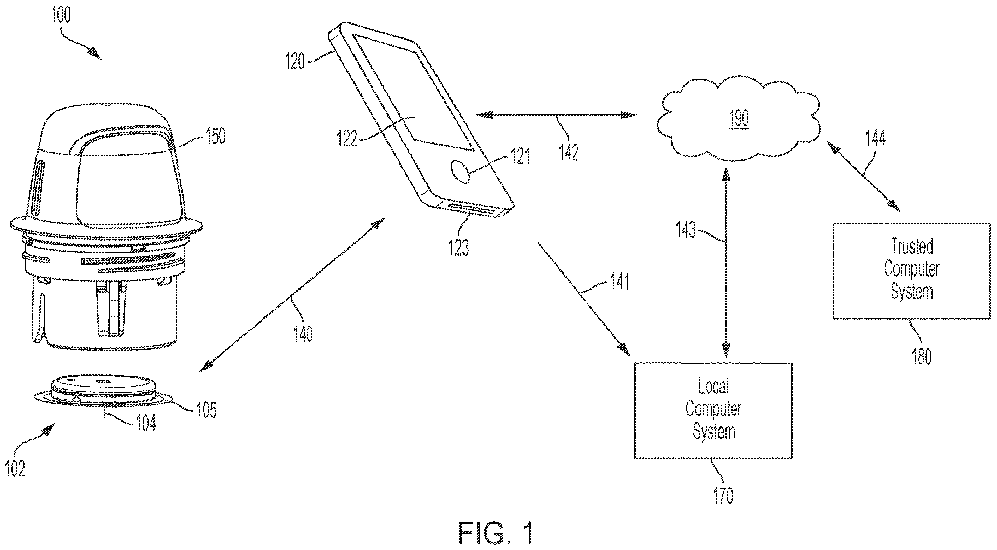

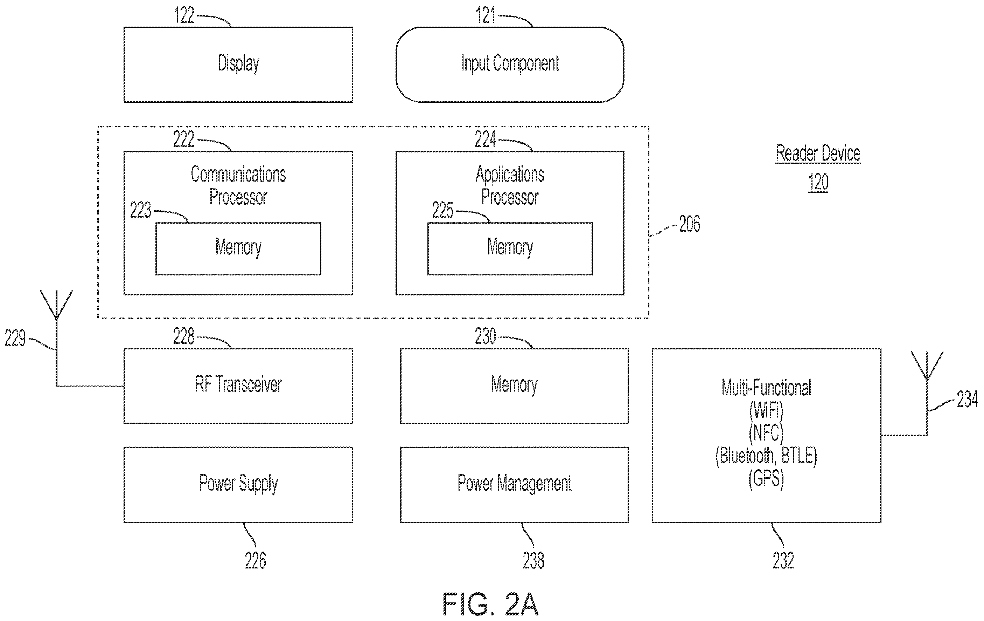

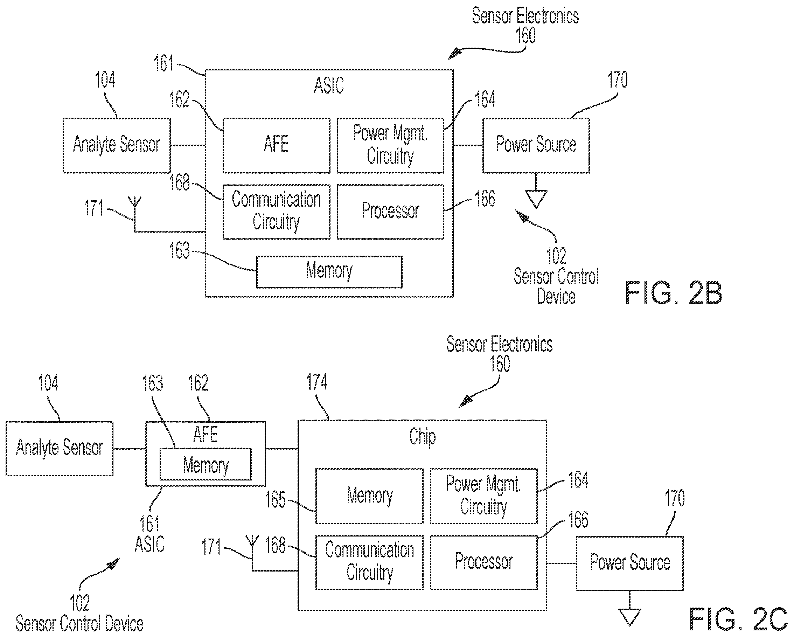

FIG. 1 is a conceptual diagram depicting an example embodiment of an analyte monitoring system 100 that includes a sensor applicator 150, a sensor control device 102, and a reader device 120. Here, sensor applicator 150 can be used to deliver sensor control device 102 to a monitoring location on a user's skin where a sensor 104 is maintained in position for a period of time by an adhesive patch 105. Sensor control device 102 is further described in FIGS. 2B and 2C, and can communicate with reader device 120 via a communication path 140 using a wired or wireless technique. Example wireless protocols include Bluetooth, Bluetooth Low Energy (BLE, BTLE, Bluetooth SMART, etc.), Near Field Communication (NFC) and others. Users can view and use applications installed in memory on reader device 120 using screen 122 (which, in many embodiments, can comprise a touchscreen), and input 121. A device battery of reader device 120 can be recharged using power port 123. While only one reader device 120 is shown, sensor control device 102 can communicate with multiple reader devices 120. Each of the reader devices 120 can communicate and share data with one another. More details about reader device 120 is set forth with respect to FIG. 2A below. Reader device 120 can communicate with local computer system 170 via a communication path 141 using a wired or wireless communication protocol. Local computer system 170 can include one or more of a laptop, desktop, tablet, phablet, smartphone, set-top box, video game console, or other computing device and wireless communication can include any of a number of applicable wireless networking protocols including Bluetooth, Bluetooth Low Energy (BTLE), Wi-Fi or others. Local computer system 170 can communicate via communications path 143 with a network 190 similar to how reader device 120 can communicate via a communications path 142 with network 190, by a wired or wireless communication protocol as described previously. Network 190 can be any of a number of networks, such as private networks and public networks, local area or wide area networks, and so forth. A trusted computer system 180 can include a server and can provide authentication services and secured data storage and can communicate via communications path 144 with network 190 by wired or wireless technique.

### Example Embodiment of Reader Device

FIG. 2A is a block diagram depicting an example embodiment of a reader device 120, which, in some embodiments, can comprise a smartphone. Here, reader device 120 can include a display 122, input component 121, and a processing core 206 including a communications processor 222 coupled with memory 223 and an applications processor 224 coupled with memory 225. Also included can be separate memory 230, RF transceiver 228 with antenna 229, and power supply 226 with power management module 238. Further, reader device 120 can also include a multi-functional transceiver 232 which can communicate over Wi-Fi, NFC, Bluetooth, BTLE, and GPS with an antenna 234. As understood by one of skill in the art, these components are electrically and communicatively coupled in a manner to make a functional device.

### Example Embodiments of Sensor Control Devices

FIGS. 2B and 2C are block diagrams depicting example embodiments of sensor control devices 102 having analyte sensors 104 and sensor electronics 160 (including analyte monitoring circuitry) that can have the majority of the processing capability for rendering end-result data suitable for display to the user. In FIG. 2B, a single semiconductor chip 161 is depicted that can be a custom application specific integrated circuit (ASIC). Shown within ASIC 161 are certain high-level functional units, including an analog front end (AFE) 162, power management (or control) circuitry 164, processor 166, and communication circuitry 168 (which can be implemented as a transmitter, receiver, transceiver, passive circuit, or otherwise according to the communication protocol). In this embodiment, both AFE 162 and processor 166 are used as analyte monitoring circuitry, but in other embodiments either circuit can perform the analyte monitoring function. Processor 166 can include one or more processors, microprocessors, controllers, and/or microcontrollers, each of which can be a discrete chip or distributed amongst (and a portion of) a number of different chips.

A memory 163 is also included within ASIC 161 and can be shared by the various functional units present within ASIC 161, or can be distributed amongst two or more of them. Memory 163 can also be a separate chip. Memory 163 can be volatile and/or non-volatile memory. In this embodiment, ASIC 161 is coupled with power source 170, which can be a coin cell battery, or the like. AFE 162 interfaces with in vivo analyte sensor 104 and receives measurement data therefrom and outputs the data to processor 166 in digital form, which in turn processes the data to arrive at the end-result glucose discrete and trend values, etc. This data can then be provided to communication circuitry 168 for sending, by way of antenna 171, to reader device 120 (not shown), for example, where minimal further processing is needed by the resident software application to display the data.

FIG. 2C is similar to FIG. 2B but instead includes two discrete semiconductor chips 162 and 174, which can be packaged together or separately. Here, AFE 162 is resident on ASIC 161. Processor 166 is integrated with power management circuitry 164 and communication circuitry 168 on chip 174. AFE 162 includes memory 163 and chip 174 includes memory 165, which can be isolated or distributed within. In one example embodiment, AFE 162 is combined with power management circuitry 164 and processor 166 on one chip, while communication circuitry 168 is on a separate chip. In another example embodiment, both AFE 162 and communication circuitry 168 are on one chip, and processor 166 and power management circuitry 164 are on another chip. It should be noted that other chip combinations are possible, including three or more chips, each bearing responsibility for the separate functions described, or sharing one or more functions for fail-safe redundancy.

### Example Embodiments of User Interfaces for Analyte Monitoring Systems

Described herein are example embodiments of GUIs for analyte monitoring systems. As an initial matter, it will be understood by those of skill in the art that the GUIs described herein comprise instructions stored in a memory of reader device 120, local computer system 170, trusted computer system 180, and/or any other device or system that is part of, or in communication with, analyte monitoring system 100. These instructions, when executed by one or more processors of the reader device 120, local computer system 170, trusted computer system 180, or other device or system of analyte monitoring system 100, cause the one or more processors to perform the method steps and/or output the GUIs described herein. Those of skill in the art will further recognize that the GUIs described herein can be stored as instructions in the memory of a single centralized device or, in the alternative, can be distributed across multiple discrete devices in geographically dispersed locations.

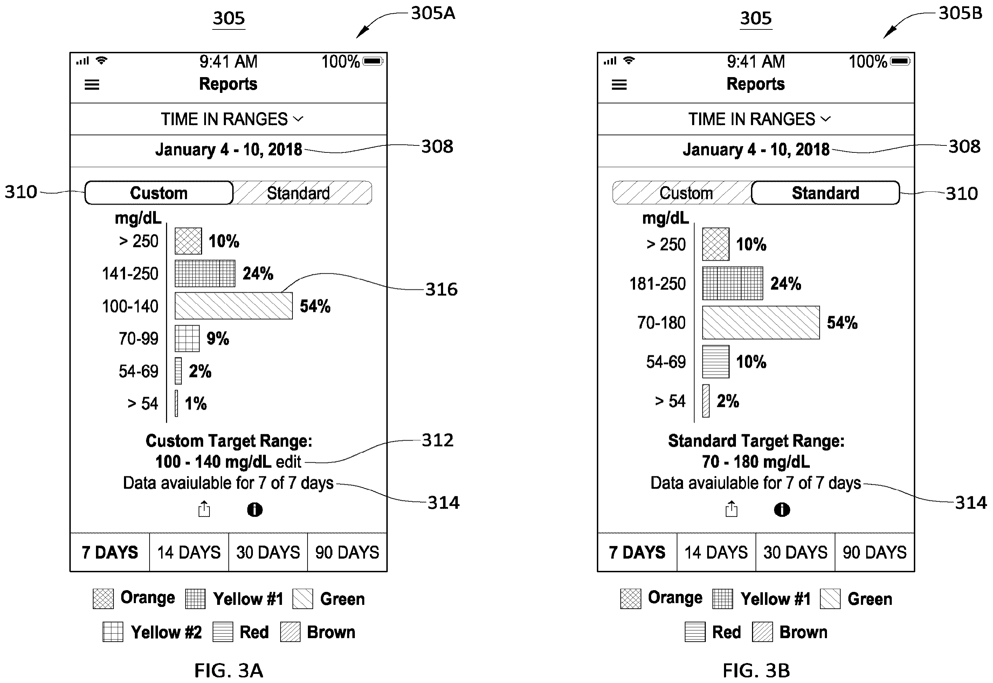

FIGS. 3A to 3F depict example embodiments of GUIs for analyte monitoring systems. In particular, FIGS. 3A to 3F depict Time-in-Ranges (also referred to as Time-in-Range and/or Time-in-Target) GUIs, each of which comprise a plurality of bars or bar portions, wherein each bar or bar portion indicates an amount of time that a user's analyte level is within a predefined analyte range correlating with the bar or bar portion. In some embodiments, for example, the amount of time can be expressed as a percentage of a predefined amount of time.

Turning to FIGS. 3A and 3B, an example embodiment of a Time-in-Ranges GUI 305 is shown, wherein Time-in-Ranges GUI 305 comprises a "Custom" Time-in-Ranges view 305A and a "Standard" Time-in-Ranges view 305B, with a toggle, switch, or slidable element 310 that allows the user to select between the two views. According to one aspect of the embodiments, Time-in-Ranges views 305A, 305B can each comprise multiple bars, wherein each bar indicates an amount of time that a user's analyte level is within a predefined analyte range correlating with the bar. In some embodiments, Time-in-Ranges views 305A, 305B further comprise a date range indicator 308, showing relevant dates associated with the displayed analyte data, and a data availability indicator 314, showing the period(s) of time in which analyte data is available for the displayed analyte data (e.g., "Data available for 7 of 7 days").

Referring to FIG. 3A, "Custom" Time-in-Ranges view 305A includes six bars comprising (from top to bottom): a first bar indicating that the user's glucose range is above 250 mg/dL for 10% of a predefined amount of time, a second bar indicating that the user's glucose range is between 141 and 250 mg/dL for 24% of the predefined amount of time, a third bar 316 indicating that the user's glucose range is between 100 and 140 mg/dL for 54% of the predefined amount of time, a fourth bar indicating that the user's glucose range is between 70 and 99 mg/dL for 9% of the predefined amount of time, a fifth bar indicating that the user's glucose range is between 54 and 69 mg/dL for 2% of the predefined amount of time, and a sixth bar indicating that the user's glucose range is less than 54 mg/dL for 1% of the predefined amount of time. Those of skill in the art will recognize that the glucose ranges and percentages of time associated with each bar can vary depending on the ranges defined by the user and the available analyte data of the user. Furthermore, although FIGS. 3A and 3B show a predefined amount of time 314 equal to seven days, those of skill in the art will appreciate that other predefined amounts of time can be utilized (e.g., one day, three days, fourteen days, thirty days, ninety days, etc.), and are fully within the scope of the present disclosure.

According to another aspect of the embodiments, "Custom" Time-in-Ranges view 305A also includes a user-definable custom target range 312 that includes an actionable "edit" link that allows a user to define and/or change the custom target range. As shown in "Custom" Time-in-Ranges view 305A, the custom target range 312 has been defined as a glucose range between 100 and 140 mg/dL and corresponds with third bar 316 of the plurality of bars.

Referring to FIG. 3B, "Standard" Time-in-Ranges view 305B includes five bars comprising (from top to bottom): a first bar indicating that the user's glucose range is above 250 mg/dL for 10% of a predefined amount of time, a second bar indicating that the user's glucose range is between 181 and 250 mg/dL for 24% of the predefined amount of time, a third bar indicating that the user's glucose range is between 70 and 180 mg/dL for 54% of the predefined amount of time, a fourth bar indicating that the user's glucose range is between 54 and 69 mg/dL for 10% of the predefined amount of time, and a fifth bar indicating that the user's glucose range is less than 54 mg/dL for 2% of the predefined amount of time. As with the "Custom" Time-in-Ranges view 305A, those of skill in the art will recognize that the percentages of time associated with each bar can vary depending on the available analyte data of the user. Unlike the "Custom" Time-in-Ranges view 305A, however, the glucose ranges shown in "Standard" view 305B cannot be adjusted by the user.

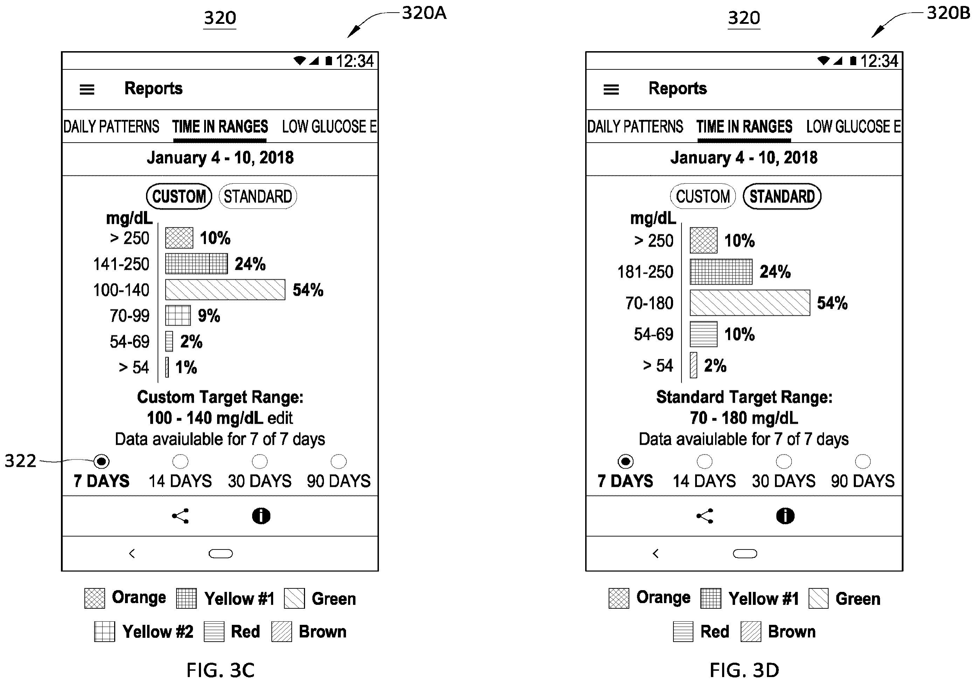

FIGS. 3C and 3D depict another example embodiment of Time-in-Ranges GUI 320 with multiple views, 320A and 320B, which are analogous to the views shown in FIGS. 3A and 3B, respectively. According to some embodiments, Time-in-Ranges GUI 320 can further include one or more selectable icons 322 (e.g., radio button, check box, slider, switch, etc.) that allow a user to select a predefined amount of time over which the user's analyte data will be shown in the Time-in-Range GUI 320. For example, as shown in FIGS. 3C and 3D, selectable icons 322 can be used to select a predefined amount of time of seven days, fourteen days, thirty days, or ninety days. Those of skill in the art will appreciate that other predefined amounts of time can be utilized and are fully within the scope of the present disclosure.

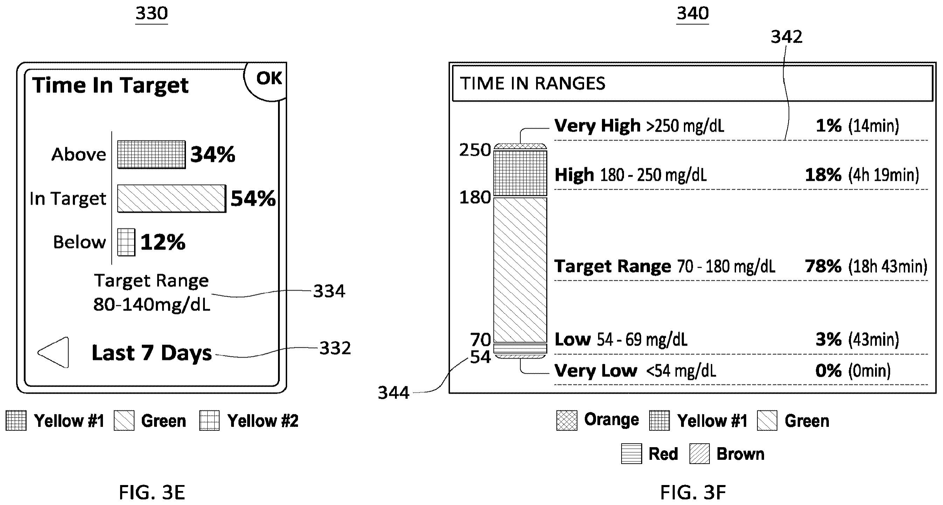

FIG. 3E depicts an example embodiment of a Time-in-Target GUI 330, which can be visually output to a display of a reader device (e.g., a dedicated reader device, a meter device, etc.). According to one aspect of the embodiments, Time-in-Target GUI 330 includes three bars comprising (from top to bottom): a first bar indicating that the user's glucose range is above a predefined target range for 34% of a predefined amount of time, a second bar indicating that the user's glucose range is within the predefined target range for 54% of the predefined amount of time, and a third bar indicating that the user's glucose range is below the predefined target range for 12% of the predefined amount of time. Those of skill in the art will recognize that the percentages of time associated with each bar can vary depending on the available analyte data of the user. Furthermore, although FIG. 3E shows a predefined amount of time 332 equal to the last seven days and a predefined target range 334 of 80 to 140 mg/dL, those of skill in the art will appreciate that other predefined amounts of time (e.g., one day, three days, fourteen days, thirty days, ninety days, etc.) and/or predefined target ranges (e.g., 70 to 180 mg/dL) can be utilized, and are fully within the scope of the present disclosure.

FIG. 3F depicts another example embodiment of a Time-in-Ranges GUI 340, which includes a single bar comprising five bar portions including (from top to bottom): a first bar portion indicating that the user's glucose range is "Very High" or above 250 mg/dL for 1% (14 minutes) of a predefined amount of time, a second bar portion indicating that the user's glucose range is "High" or between 180 and 250 mg/dL for 18% (4 hours and 19 minutes) of the predefined amount of time, a third bar portion indicating that the user's glucose range is within a "Target Range" or between 70 and 180 mg/dL for 78% (18 hours and 43 minutes) of the predefined amount of time, a fourth bar portion indicating that the user's glucose range is "Low" or between 54 and 69 mg/dL for 3% (43 minutes) of the predefined amount of time, and a fifth bar portion indicating that the user's glucose range is "Very Low" or less than 54 mg/dL for 0% (0 minutes) of the predefined amount of time.

According to one aspect of the embodiment shown in FIG. 3F, each bar portion of Time-in-Ranges GUI 340 can comprise a different color. In some embodiments, bar portions can be separated by dashed or dotted lines 342 and/or interlineated with numeric markers 344 to indicate the ranges reflected by the adjacent bar portions. In some embodiments, the time in ranges reflected by the bar portions can be further expressed as a percentage, an actual amount of time (e.g., 4 hours and 19 minutes), or, as shown in FIG. 3F, both. Furthermore, those of skill in the art will recognize that the percentages of time associated with each bar portion can vary depending on the analyte data of the user. In some embodiments of Time-in-Ranges GUI 340, the Target Range can be configured by the user. In other embodiments, the Target Range of Time-in-Ranges GUI 340 is not modifiable by the user.

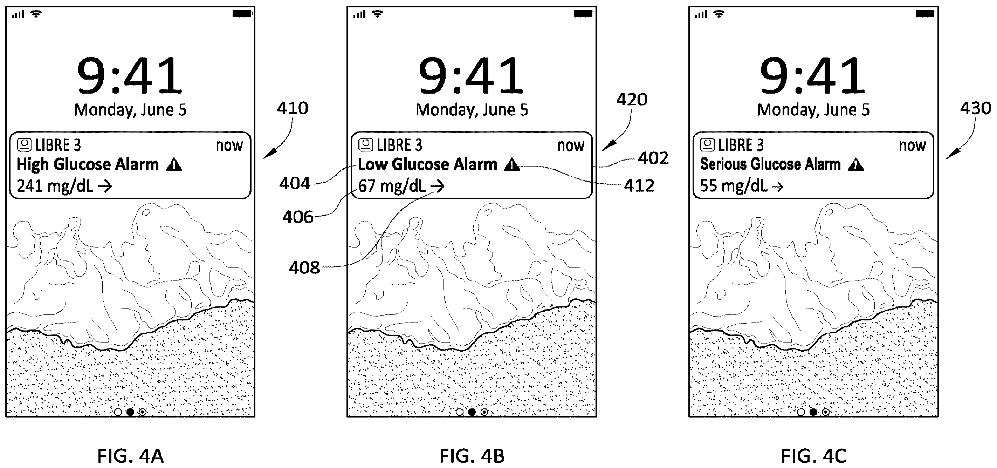

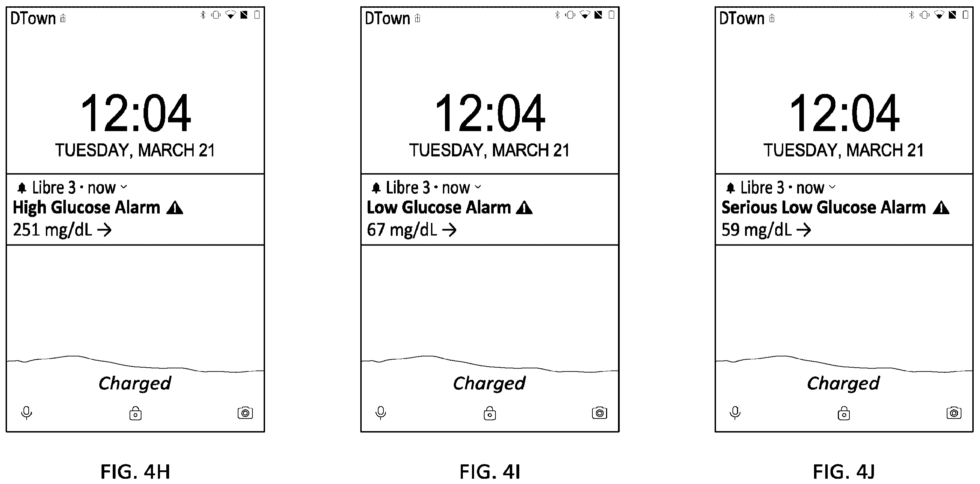

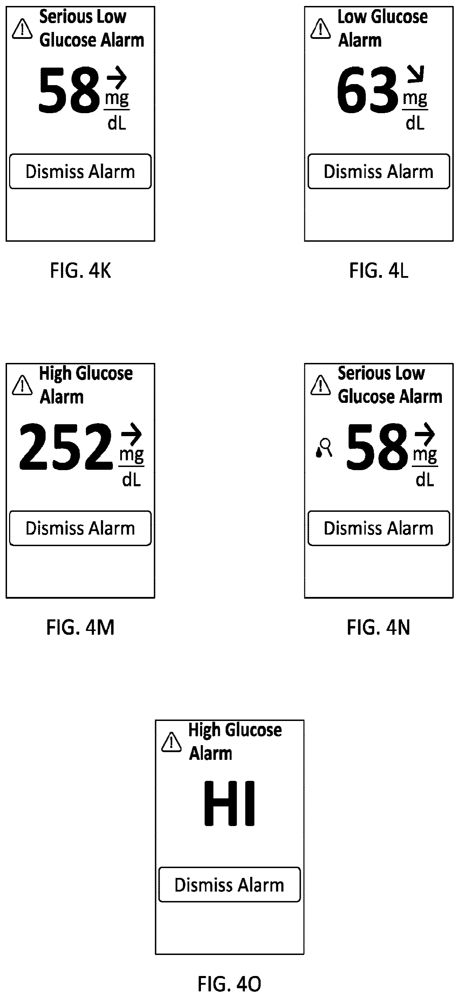

FIGS. 4A to 4O depict example embodiments of Analyte Level/Trend Alert GUIs for analyte monitoring systems. According to one aspect of the embodiments, the Analyte Level/Trend Alert GUIs comprise a visual notification (e.g., alert, alarm, pop-up window, banner notification, etc.), wherein the visual notification includes an alarm condition, an analyte level measurement associated with the alarm condition, and a trend indicator associated with the alarm condition.

Turning to FIGS. 4A to 4C, example embodiments of a High Glucose Alarm 410, Low Glucose Alarm 420, and a Serious Low Glucose Alarms 430 (sometimes, also referred to as "an Urgent Low Glucose Alarm") are depicted, respectively, wherein each alarm comprises a pop-up window 402 containing an alarm condition text 404 (e.g., "Low Glucose Alarm"), an analyte level measurement 406 (e.g., 67 mg/dL) associated with the alarm condition, and a trend indicator 408 (e.g., trend arrow) associated with the alarm condition. In some embodiments, an alarm icon 412 can be adjacent to the alarm condition text 404.

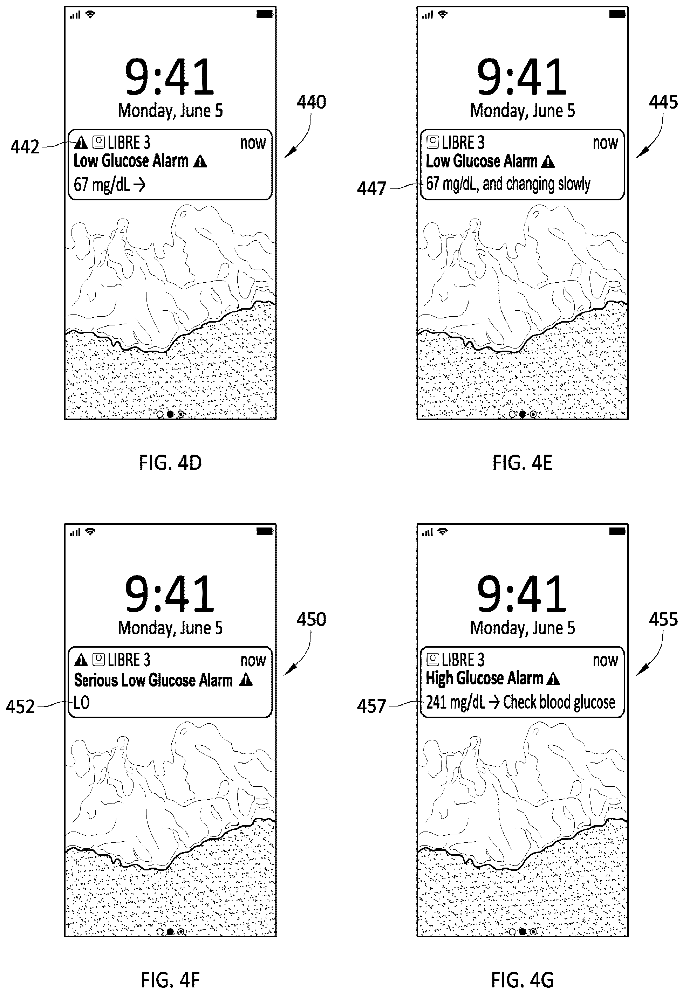

Referring next to FIGS. 4D to 4G, additional example embodiments of Low Glucose Alarms 440, 445, Serious Low Glucose Alarm 450, and High Glucose Alarm 455 are depicted, respectively. As shown in FIG. 4D, Low Glucose Alarm 440 is similar to the Low Glucose Alarm of FIG. 4B (e.g., comprises a pop-up window containing an alarm condition text, an analyte level measurement associated with the alarm condition, and a trend indicator associated with the alarm condition), but further includes a critical alert icon 442 to indicate that the alarm has been configured as a critical alert (e.g., will display, play a sound, vibrate, even if the device is locked or if the device's "Do Not Disturb" setting has been enabled). With respect to FIG. 4E, Low Glucose Alarm 445 is also similar to the Low Glucose Alarm of FIG. 4B, but instead of including a trend arrow, Log Glucose Alarm 445 includes a textual trend indicator 447. According to one aspect of some embodiments, textual trend indicator 447 can be enabled through a device's Accessibility settings such that the device will "read" the textual trend indicator 447 to the user via the device's text-to-speech feature (e.g., Voiceover for iOS or Select-to-Speak for Android).

Referring next to FIG. 4F, Low Glucose Alarm 450 is similar to the Low Glucose Alarm of FIG. 4D (including the critical alert icon), but instead of displaying an analyte level measurement associated with an alarm condition and a trend indicator associated with the alarm condition, Low Glucose Alarm 450 displays a out-of-range indicator 452 to indicate that the current glucose level is either above or below a predetermined reportable analyte level range (e.g., "HI" or "LO"). With respect to FIG. 4G, High Glucose Alarm 455 is similar to the High Glucose Alarm of FIG. 4A (e.g., comprises a pop-up window containing an alarm condition text, an analyte level measurement associated with the alarm condition, and a trend indicator associated with the alarm condition), but further includes an instruction to the user 457. In some embodiments, for example, the instruction can be a prompt for the user to "Check blood glucose." Those of skill in the art will appreciate that other instructions or prompts can be implemented (e.g., administer a corrective bolus, eat a meal, etc.).

Furthermore, although FIGS. 4A to 4G depict example embodiments of Analyte Level/Trend Alert GUIs that are displayed on smart phones having an iOS operating system, those of skill in the art will also appreciate that the Analyte Level/Trend Alert GUIs can be implemented on other devices including, e.g., smart phones with other operating systems, smart watches, wearables, reader devices, tablet computing devices, blood glucose meters, laptops, desktops, and workstations, to name a few. FIGS. 4H to 4J, for example, depict example embodiments of a High Glucose Alarm, Low Glucose Alarm, and a Serious Low Glucose Alarm for a smart phone having an Android Operating System. Similarly, FIGS. 4K to 4O depict, respectively, example embodiments of a Serious Low Glucose Alarm, Low Glucose Alarm, High Glucose Alarm, Serious Low Glucose Alarm (with a Check Blood Glucose icon), and High Glucose Alarm (with an out-of-range indicator) for a reader device. As described above, the Serious Low Glucose Alarm is sometimes also referred to as an Urgent Low Glucose Alarm, and can display the text "Urgent Low Glucose Alarm" in place of "Serious Low Glucose Alarm."

It should be noted that all features, elements, components, functions, and steps described with respect to any embodiment provided herein are intended to be freely combinable and substitutable with those from any other embodiment. If a certain feature, element, component, function, or step is described with respect to only one embodiment, then it should be understood that that feature, element, component, function, or step can be used with every other embodiment described herein unless explicitly stated otherwise. This paragraph therefore serves as antecedent basis and written support for the introduction of claims, at any time, that combine features, elements, components, functions, and steps from different embodiments, or that substitute features, elements, components, functions, and steps from one embodiment with those of another, even if the following description does not explicitly state, in a particular instance, that such combinations or substitutions are possible. It is explicitly acknowledged that express recitation of every possible combination and substitution is overly burdensome, especially given that the permissibility of each and every such combination and substitution will be readily recognized by those of ordinary skill in the art.

While the embodiments are susceptible to various modifications and alternative forms, specific examples thereof have been shown in the drawings and are herein described in detail. It should be understood, however, that these embodiments are not to be limited to the particular form disclosed, but to the contrary, these embodiments are to cover all modifications, equivalents, and alternatives falling within the spirit of the disclosure. Furthermore, any features, functions, steps, or elements of the embodiments may be recited in or added to the claims, as well as negative limitations that define the inventive scope of the claims by features, functions, steps, or elements that are not within that scope.

The invention is defined in the appended claims. A non-exhaustive list of aspects of the disclosure set out in the numbered clauses is useful for understanding the invention:
Clause 1: An analyte monitoring system, comprising: a sensor control device comprising an analyte sensor and sensor electronics, wherein the sensor control device is configured to transmit data indicative of an analyte level; and a reader device comprising a display, a transceiver configured to receive the data indicative of the analyte level, and a memory coupled with one or more processors, wherein the memory is configured to store instructions that, when executed by the one or more processors, cause the one or more processors to output to the display a plurality of bars, wherein each bar indicates an amount of time that a user's analyte level is within a predefined analyte range associated with each bar, wherein the plurality of bars is based on the data indicative of the analyte level.
Clause 2: The analyte monitoring system of clause 1, wherein the amount of time comprises a percentage of a predefined time period.
Clause 3: The analyte monitoring system of clause 1, wherein the data indicative of the analyte level comprises data indicative of a glucose level in a bodily fluid.
Clause 4: The analyte monitoring system of clause 1, wherein the plurality of bars is a first plurality of bars, wherein the instructions, when executed by the one or more processors, further cause the one or more processors to output to the display a second plurality of bars, wherein each of the second plurality of bars indicates an amount of time that a user's analyte level is within a predefined analyte range associated with each bar, wherein the second plurality of bars is based on the data indicative of the analyte level, and wherein the first plurality of bars is customizable by the user and the second plurality of bars is not customizable by the user.
Clause 5: The analyte monitoring system of clause 4, wherein the instructions, when executed by the one or more processors, further cause the one or more processors to output to the display a slidable element configured to allow the user to select between displaying the first plurality of bars or the second plurality of bars on the display.
Clause 6: The analyte monitoring system of clause 1, wherein the instructions, when executed by the one or more processors, further cause the one or more processors to output to the display a date range indicator comprising a date range associated with the plurality of bars and the data indicative of the analyte level.
Clause 7: The analyte monitoring system of clause 1, wherein the instructions, when executed by the one or more processors, further cause the one or more processors to output to the display a data availability indicator comprising a period of time in which the data indicative of the analyte level is available.
Clause 8: The analyte monitoring system of clause 1, wherein at least one predefined analyte range associated with the plurality of bars is adjustable by the user.
Clause 9: The analyte monitoring system of clause 4, wherein none of the predefined analyte ranges associated with the second plurality of bars is adjustable by the user.
Clause 10: The analyte monitoring system of clause 1, wherein the instructions, when executed by the one or more processors, further cause the one or more processors to output to the display a plurality of selectable icons configured to allow a user to select a predefined amount of time associated with the data indicative of the analyte level.
Clause 11. An analyte monitoring system, comprising: a display; and one or more processors coupled with a memory, the memory configured to store instructions that, when executed by the one or more processors, cause the one or more processors to output to the display a bar comprising a plurality of bar portions, wherein each bar portion of the plurality of bar portions indicates an amount of time that a user's analyte level is within a predefined analyte range associated with each bar portion, and wherein the plurality of bar portions are based on data indicative of an analyte level.
Clause 12: The analyte monitoring system of clause 11, wherein the amount of time comprises a percentage of a predefined time period and an actual amount of time.
Clause 13: The analyte monitoring system of clause 11, wherein the data indicative of the analyte level comprises data indicative of a glucose level in a bodily fluid.
Clause 14: The analyte monitoring system of clause 11, wherein the one or more processors comprise one or more processors of a cloud-based platform.
Clause 15: The analyte monitoring system of clause 11, wherein each of the bar portions comprises a different color.
Clause 16: An analyte monitoring system, comprising: a sensor control device comprising an analyte sensor coupled with sensor electronics, the sensor control device configured to transmit data indicative of an analyte level; and a reader device comprising a display, wireless communication circuitry configured to receive the data indicative of the analyte level, one or more processors coupled with a memory, the memory configured to store instructions that, when executed by the one or more processors, cause the one or more processors to output to the display an alert interface comprising an alarm condition, an analyte level measurement associated with the alarm condition, and a trend indicator associated with the alarm condition.
Clause 17: The analyte monitoring system of clause 16, wherein the alarm condition is one of a low glucose condition, a serious low glucose condition, or a high glucose condition.
Clause 18: The analyte monitoring system of clause 16, wherein alert interface further comprises an alarm icon adjacent to the alarm condition.
Clause 19: The analyte monitoring system of clause 18, wherein the alarm icon is a critical alert icon.
Clause 20: The analyte monitoring system of clause 16, wherein the alert interface is a pop-up window.
Clause 21: The analyte monitoring system of clause 16, wherein the alert interface is a banner notification.
Clause 22: The analyte monitoring system of clause 16, wherein the trend indicator is a directional arrow.
Clause 23: The analyte monitoring system of clause 16, wherein the trend indicator is a textual trend indicator, and wherein the instructions, when executed by the one or more processors, further cause the one or more processors to read the textual trend indicator using a text-to-speech feature.
Clause 24: The analyte monitoring system of clause 16, wherein the analyte level measurement is a current glucose level.
Clause 25: The analyte monitoring system of clause 16, wherein the alert interface further comprises an instruction to the user.
Clause 26: The analyte monitoring system of clause 25, wherein the instruction to the user comprises one of an instruction to check blood glucose, an instruction to administer medication, or an instruction to eat a meal.
Clause 27: The analyte monitoring system of clause 16, wherein the reader device is a smart phone.
Clause 28: An analyte monitoring system, comprising: a sensor control device comprising an analyte sensor coupled with sensor electronics, the sensor control device configured to transmit data indicative of an analyte level; and a reader device comprising a display, wireless communication circuitry configured to receive the data indicative of the analyte level, one or more processors coupled with a memory, the memory configured to store instructions that, when executed by the one or more processors, cause the one or more processors to output to the display an alert interface comprising an alarm condition, an out-of-range indicator associated with the alarm condition, and a trend indicator associated with the alarm condition.
Clause 29: The analyte monitoring system of clause 28, wherein the out-of-range indicator comprises one of a high out-of-range indicator or a low out-of-range indicator.
Clause 30: The analyte monitoring system of clause 28, wherein the reader device is a smart phone.
Clause 31: An analyte monitoring system, comprising: an on-body unit comprising an analyte sensor and sensor electronics, wherein the on-body unit is configured to transmit data indicative of an analyte level of a user; and a reader device comprising a display, a transceiver configured to receive the data indicative of the analyte level, and a memory coupled with one or more processors, wherein the memory is configured to store instructions that, when executed by the one or more processors, cause the one or more processors to: output to the display a first view comprising a first set of graphical elements, wherein each graphical element of the first set is representative of an amount of time that the analyte level of the user is within a predefined analyte range associated with a correlating graphical element of the first set, and wherein at least one of the graphical elements of the first set is customizable by the user, and output to the display a second view comprising a second set of graphical elements, wherein each graphical element of the second set is representative of an amount of time that the analyte level of the user is within a predefined analyte range associated with a correlating graphical element of the second set, and wherein the graphical elements of the second set are not customizable by the user.
Clause 32: The analyte monitoring system of clause 31, wherein the instructions, when executed by the one or more processors, further cause the one or more processors to: output to the display a switch element, wherein the switch is configured to cause the first view or the second view to output to the display of the reader device.
Clause 33: The analyte monitoring system of clause 32, wherein the switch element comprises a toggle.
Clause 34: The analyte monitoring system of clause 32, wherein the switch element comprises a slidable element.
Clause 35: The analyte monitoring system of clause 31, wherein the at least one of the graphical elements of the first set that is customizable by the user comprises a customizable target analyte range.
Clause 36: The analyte monitoring system of clause 31, wherein each of the graphical elements of the first set comprises a different color from the other graphical elements of the first set.
Clause 37: The analyte monitoring system of clause 31, wherein each of the graphical elements of the second set comprises a different color from the other graphical elements of the second set.
Clause 38: The analyte monitoring system of clause 31, wherein a total number of graphical elements of the first set is equal to a total number of graphical elements of the second set.
Clause 39: The analyte monitoring system of clause 31, wherein a total number of graphical elements of the first set is not equal to a total number of graphical elements of the second set.
Clause 40: The analyte monitoring system of clause 31, wherein the reader device comprises a smart phone.
Clause 41: The analyte monitoring system of clause 31, wherein the reader device comprises a smart watch.
Clause 42: The analyte monitoring system of clause 31, wherein the amount of time that the analyte level of the user is within the predefined analyte range comprises a percentage of a predefined time period.
Clause 43: The analyte monitoring system of clause 31, wherein the data indicative of the analyte level comprises data indicative of a glucose level in a bodily fluid of the user.
Clause 44: The analyte monitoring system of clause 31, wherein the instructions, when executed by the one or more processors, further cause the one or more processors to output to the display a date range indicator comprising a date range associated with the first set of graphical elements and the second set of graphical elements.
Clause 45: The analyte monitoring system of clause 31, wherein the instructions, when executed by the one or more processors, further cause the one or more processors to output to the display a data availability indicator comprising a period of time in which the data indicative of the analyte level is available.
Clause 46: The analyte monitoring system of clause 31, wherein the instructions, when executed by the one or more processors, further cause the one or more processors to output to the display a plurality of selectable icons configured to allow a user to select a predefined amount of time associated with the data indicative of the analyte level.
Clause 47: The analyte monitoring system of clause 35, wherein the instructions, when executed by the one or more processors, further cause the one or more processors to output to the display a link to an interface configured to allow the user to edit the customizable target analyte range.

## Claims

1. An analyte monitoring system, comprising:
a sensor control device comprising an analyte sensor and sensor electronics, wherein the sensor control device is configured to transmit data indicative of an analyte level; and
a reader device comprising a display, a transceiver configured to receive the data indicative of the analyte level, and a memory coupled with one or more processors, wherein the memory is configured to store instructions that, when executed by the one or more processors, cause the one or more processors to output to the display a plurality of bars, wherein each bar indicates an amount of time that a user's analyte level is within a predefined analyte range associated with each bar, wherein the plurality of bars is based on the data indicative of the analyte level, wherein
the plurality of bars is a first plurality of bars, wherein the instructions, when executed by the one or more processors, further cause the one or more processors to output to the display a second plurality of bars,
wherein each of the second plurality of bars indicates an amount of time that a user's analyte level is within a predefined analyte range associated with each bar, wherein the second plurality of bars is based on the data indicative of the analyte level, and
wherein the first plurality of bars is customizable by the user and the second plurality of bars is not customizable by the user.

2. The analyte monitoring system of claim 1, wherein the amount of time comprises a percentage of a predefined time period.

3. The analyte monitoring system of claim 1, wherein the data indicative of the analyte level comprises data indicative of a glucose level in a bodily fluid.

4. The analyte monitoring system of claim 1, wherein the instructions, when executed by the one or more processors, further cause the one or more processors to output to the display a slidable element configured to allow the user to select between displaying the first plurality of bars or the second plurality of bars on the display.

5. The analyte monitoring system of claim 1, wherein the instructions, when executed by the one or more processors, further cause the one or more processors to output to the display a date range indicator comprising a date range associated with the plurality of bars and the data indicative of the analyte level.

6. The analyte monitoring system of claim 1, wherein the instructions, when executed by the one or more processors, further cause the one or more processors to output to the display a data availability indicator comprising a period of time in which the data indicative of the analyte level is available.

7. The analyte monitoring system of claim 1, wherein at least one predefined analyte range associated with the plurality of bars is adjustable by the user.

8. The analyte monitoring system of claim 1, wherein none of the predefined analyte ranges associated with the second plurality of bars is adjustable by the user.

9. The analyte monitoring system of claim 1, wherein the instructions, when executed by the one or more processors, further cause the one or more processors to output to the display a plurality of selectable icons configured to allow a user to select a predefined amount of time associated with the data indicative of the analyte level.

10. An analyte monitoring system, comprising:
a display; and
one or more processors coupled with a memory, the memory configured to store instructions that, when executed by the one or more processors, cause the one or more processors to output to the display a bar comprising a plurality of bar portions,
wherein each bar portion of the plurality of bar portions indicates an amount of time that a user's analyte level is within a predefined analyte range associated with each bar portion, and wherein the plurality of bar portions are based on data indicative of an analyte level.

11. The analyte monitoring system of claim 10, wherein the amount of time comprises a percentage of a predefined time period and an actual amount of time.

12. The analyte monitoring system of claim 10, wherein the data indicative of the analyte level comprises data indicative of a glucose level in a bodily fluid.

13. The analyte monitoring system of claim 10, wherein the one or more processors comprise one or more processors of a cloud-based platform.

14. The analyte monitoring system of claim 10, wherein each of the bar portions comprises a different color.
